# EUROPÄISCHE PATENTANMELDUNG

(11) **EP 2 786 774 A1**
(43) Veröffentlichungstag der Anmeldung: **08.10.2014**
(21) Anmeldenummer: 13162267.2
(22) Anmeldetag: 04.04.2013
(51) Int. Cl.: A61M 5/28

(54) **Einwegspritze**

(71) Anmelder: Flamina Holding AG, 6304 Zug (CH)
(72) Erfinder: May, Michael, 6304 Zug (CH)
(74) Vertreter: Rutz & Partner

(57) **Zusammenfassung**

Die Einwegspritze (1) weist einen Spritzenkörper (10), der einen der Aufnahme einer Flüssigkeit (2) dienenden Behälter (110) umfasst, und eine Injektionsnadel (13) auf, durch die hindurch die Flüssigkeit (2) aus dem Behälter (110) transferierbar ist. Erfindungsgemäss weist der Spritzenkörper (10) ein erstes Rahmenteil (11) auf, das mit dem manuell komprimierbaren Behälter (110) versehen ist und das über wenigstens eine Sollbruchstelle (18) einstückig mit einem zweiten Rahmenteil (12) verbunden ist, welches eine Ausnehmung (120) aufweist, in die die Injektionsnadel (13) hinein ragt, deren dem Behälter (11) zugewandtes Endstück (130) vom ersten Rahmenteil (11) dicht abschliessend gehalten ist.

## Beschreibung

Die Erfindung betrifft eine für den einmaligen Gebrauch bestimmte Injektionsspritze bzw. eine Fertigspritze oder Einwegspritze (engl.: disposable syringe), mittels der ein bestimmtes Volumen eines flüssigen Medikaments einem Empfänger zugeführt werden kann.

Einwegspritzen gelangen üblicherweise dann zum Einsatz, wenn eine Applikation eines Medikaments durch den Patienten selbst erfolgen soll. Typische Beispiele sind Diabetesspritzen oder Spritzen für Asthmatiker, bei denen eine subkutane Anwendung eines Medikaments erforderlich ist, falls der Asthmaspray versagen sollte. Einwegspritzen gehören üblicherweise auch zur Ausrüstung von Personen, die in militärischem Einsatz stehen oder Personen, die im Umfeld hochgiftiger Chemikalien arbeiten. Einwegspritzen gehören daher zur Notfallausrüstung in verschiedenen Einsatzgebieten.

Injektionsspritzen und auch Einwegspritzen, wie sie aus der Offenlegungsschrift DE102007047346A1 bekannt sind, weisen üblicherweise einen Kolben auf, der in einen Hohlzylinder einführbar ist, um eine darin vorgesehene medizinische Flüssigkeit durch eine Kanüle oder Injektionsnadel nach aussen zu verdrängen. Die Länge der heute verwendeten Injektionsnadeln, die sehr dünn sind, liegt üblicherweise im Bereich von 6mm bis 12mm. Einwegspritzen dieser Art weisen insbesondere bei der Anwendung in Notfallsituationen erhebliche Nachteile auf.

Die Vorbereitung der Einwegspritzen für den Einsatz nimmt oft wertvolle Zeit in Anspruch. Die Handhabung der Einwegspritze erfordert zudem Geschick, weshalb insbesondere in Notfallsituationen der Einsatz der Einwegspritzen fehlschlagen kann. Weiterhin nehmen Einwegspritzen relativ viel Raum in Anspruch, weshalb diese in gesonderten Taschen oder Etuis mitgetragen werden müssen. Weiterhin können mechanische Einwirkungen zu einer Beschädigung der bekannten Einwegspritzen führen, weshalb diese im Notfall ggf. Nicht mehr verwendbar sind.

Der vorliegenden Erfindung liegt daher die Aufgabe zugrunde, eine verbesserte Einwegspritze zu schaffen.

Insbesondere ist eine Einwegspritze zu schaffen, die mit wenigen einfachen Handgriffen zum Einsatz gelangen kann. Die Einwegspritze soll leicht handhabbar sein und auch mit nur einer Hand eingesetzt werden können. Die medizinische Flüssigkeit soll mit geringer Kraft durch die Injektionsnadel ausgestossen werden können.

Die Einwegspritze soll zudem robust aufgebaut sein, so dass auch bei stärkeren mechanischen Einwirkungen keine Beeinträchtigung der Funktionen oder der Dichtigkeit der Einwegspritze erfolgt und die Sterilität der medizinischen Flüssigkeit gewährleistet bleibt.

Ferner soll die Einwegspritze nur wenig Raum in Anspruch nehmen, so dass sie beispielsweise auch in einer Brieftasche oder an einem Armband angeordnet werden kann, auf das der Anwender sofort zugreifen kann.

Die Einwegspritze soll zudem mit geringem Aufwand und mit geringen Kosten hergestellt und ausgeliefert werden können.

Diese Aufgabe wird mit einer Einwegspritze gelöst, welche die in Anspruch 1 angegebenen Merkmale aufweist. Vorteilhafte Ausgestaltungen der Erfindung sind in weiteren Ansprüchen angegeben.

Die Einwegspritze weist einen Spritzenkörper auf, der einen der Aufnahme einer Flüssigkeit dienenden Behälter umfasst. Weiter ist eine Injektionsnadel vorgesehen, durch die hindurch die Flüssigkeit aus dem Behälter in den subkutanen Anwendungsbereich transferierbar ist.

Erfindungsgemäss weist der Spritzenkörper ein erstes Rahmenteil auf, das mit dem Behälter versehen ist, welcher derart ausgestaltet ist, dass er durch manuelle Krafteinwirkung komprimierbar ist. Das erste Rahmenteil ist über wenigstens eine Sollbruchstelle bzw. Material, das wenigstens eine Sollbruchstelle aufweist, einstückig mit einem zweiten Rahmenteil verbunden, welches eine Ausnehmung aufweist, in die die Injektionsnadel hinein ragt, deren dem Behälter zugewandtes Endstück vom ersten Rahmenteil dicht abschliessend gehalten ist. Die im Behälter gespeicherte Flüssigkeit kann daher nur durch die Injektionsnadel aus dem Behälter abgeführt werden.

Auf die Verwendung eines Kolbens wird verzichtet, weshalb mit der erfindungsgemässen Ausgestaltung der Einwegspritze zahlreiche Vorteile resultieren.

Die Einwegspritze ist einfach aufgebaut und weist nur einfach ausgestaltete Vorrichtungsteile auf, die mit unkritischen Fertigungstoleranzen hergestellt und miteinander verbunden werden können. Aufgrund des Verzichts auf einen Kolben resultieren zudem geringe Abmessungen. Die kleinen und leichten Einwegspritzen können vom Anwender daher bequem und unauffällig mitgetragen werden.

Die erfindungsgemässe Einwegspritze ist zudem sehr robust und verträgt auch mechanische Einwirkungen, die zu Verbiegungen führen und für eine Kolbenspitze kritisch wären.

Die Injektionsnadel ist fest mit der Einwegspritze verbunden und muss nicht erst aufgesetzt werden. Die Injektionsnadel ist im ersten Rahmenteil, anschliessend an den Behälter oder in diesen hinein ragend, dicht umschlossen gehalten, so dass die Flüssigkeit nur durch die Injektionsnadel aus dem Behälter entweichen kann.

Zum Schutz der Injektionsnadel ist das zweite Rahmenteil vorgesehen, welches eine Ausnehmung aufweist, in die die Injektionsnadel hinein ragt. Die Injektionsnadel ist daher innerhalb des zweiten Rahmenteils sicher gehalten und gegen mechanische und gegen bakterielle Einwirkungen geschützt.

Das erste und das zweite Rahmenteil sind vorzugsweise durch ein Verbindungselement, wie einen oder mehrere Stege oder durch einen frontseitig abgeschlossenen Tunnel, einstückig miteinander verbunden. Das Verbindungselement ist mit der wenigstens einen Sollbruchstelle versehen und begrenzt die der Aufnahme der Injektionsnadel dienenden Ausnehmung zumindest teilweise oder vollständig. Vorzugsweise wird ein Tunnel vorgesehen, welcher sich vom Behälter bis zum Ende des zweiten Rahmenteils erstreckt und den aus dem Behälter hervorragenden Teil der Injektionsnadel vollständig umschliesst und schützt.

Vorzugsweise ist die Injektionsnadel zusätzlich mit einer Schutzhülle versehen, welche der Injektionsnadel weitere Stabilität verleiht und diese gegebenenfalls abschliesst. Vorzugsweise wird eine starre Schutzhülle vorgesehen, die kraftschlüssig oder formschlüssig mit dem ersten Rahmenteil verbunden ist oder verbunden sein kann.

Durch die Schutzhülle, die im genannten Tunnel gehalten ist, wird die Injektionsnadel ferner gegen das zweite Rahmenteil isoliert, so dass die Injektionsnadel beim Fertigungsvorgang nicht mit dem zweiten Rahmenteil verkleben kann. Die Schutzhülle kann mit dem zweiten Rahmenteil vergossen bzw. zwischen den beiden Schalen des zweiten Rahmenteils in einer Ausnehmung bzw. im Tunnel eingegossen werden. Mit der Verdrehung des zweiten Rahmenteils gegenüber dem ersten Rahmenteil um die Längsachse der Einwegspritze kann das zweite Rahmenteil zusammen mit der Schutzhülle vom ersten Rahmenteil und von der Injektionsnadel getrennt werden.

In vorzugsweisen Ausgestaltungen ist der Spritzenkörper zusätzlich von einer Metallfolie oder einer Kunststofffolie umschlossen, welche der Einwegspritze zusätzlichen Schutz verleiht. Diese Folie wird vorzugsweise mit einem Band versehen, welches erlaubt, die Folie aufzureissen und zu entfernen.

Durch die Anordnung wenigstens einer Sollbruchstelle, die das vorzugsweise tunnelförmige Verbindungselement durchläuft, kann das zweite Rahmenteil durch eine Drehung mit geringem Kraftaufwand vom ersten Rahmenteil gelöst und die Einwegspritze für die Anwendung vorbereitet werden.

Im Bereich der wenigstens einen Sollbruchstelle sind vorzugsweise seitliche Einschnitte oder Einrisse vorgesehen, weshalb die Sollbruchstelle durch eine Drehbewegung leicht aufgetrennt werden kann. Beim sonstigen Auftreten von Zugkräften oder Biegekräften wird die Sollbruchstelle hingegen nicht geschwächt, so dass ein selbsttätiges Lösen des zweiten Rahmenteils während des Transports der Einwegspritze verhindert wird. Die Einwegspritzen können vom Anwender daher in einer Kleidungstasche ohne spezielles Schutzetui mitgetragen werden. Aufgrund des geringen Gewichts und der geringen Abmessungen können die Einwegspritzen auch bequem an einem elastischen Band mitgetragen werden.

Die wenigstens eine Sollbruchstelle weist vorzugsweise Materialverdünnungen und/oder Perforation auf, so dass auch widerstandfähiges Material aufgetrennt werden kann. Ferner sind zwischen dem ersten und dem zweiten Rahmenteil vorzugsweise tiefere Einschnitte vorhanden, so dass sich der Querschnitt der Sollbruchstelle auf einen Bruchteil des Querschnitts der Rahmenteile reduziert.

Nach dem Entfernen des zweiten Rahmenteils besteht die Einwegspritze noch aus dem ersten Rahmenteil mit dem Behälter und der damit verbundenen Injektionsnadel. Die Injektionsnadel kann nun in einen Körperteil eingeführt werden, wonach der Behälter, der aus elastischem Material oder einer Folie besteht, durch beidseitigen manuellen Druck komprimiert wird. Die Flüssigkeit wird aus dem Behälter verdrängt und kann durch die Injektionsnadel subkutan in den Körperteil eindringen. Besonders leicht lässt sich der Behälter komprimieren, wenn dieser zylindrisch ausgestaltet ist.

Damit die Flüssigkeit gleichmässig und vollständig aus dem Behälter ausgepresst werden kann, ist dieser vorzugsweise beidseitig mit vorzugsweise balkenförmigen starren Greifelementen versehen, welche sich vorzugsweise über die gesamte Länge des Behälters erstrecken. Die beiden Greifelemente werden gegeneinander gedrückt, bis sie auf einander auftreffen. Auf diese Weise ist eine bequeme und sichere Bedienung der Einwegspritze auch mit nur einer Hand möglich. Die Einwegspritze kann somit innerhalb von wenigen Sekunden für den Einsatz vorbereitet und auch bequem und sicher eingesetzt werden.

In vorzugsweisen Ausgestaltungen weisen das erste und/oder das zweite Rahmenteil durch Einformungen gebildete oder farbliche Kennzeichen auf, anhand denen der Anwender optisch oder durch Abtasten erkennt, welche Füllung die Einwegspritze enthält.

Zur Fertigung der erfindungsgemässen Einwegspritzen werden vorzugsweise zwei zueinander komplementäre und vorzugsweise identische Schalen vorgesehen, die miteinander verbunden werden. Beispielsweise werden die beiden Schalen miteinander verschweisst, verleimt oder vergossen. Für die Fertigung der Einwegspritzen wird vorzugsweise hartelastisches Kunststoffmaterial verwendet. Zur Erhöhung der Stabilität werden das erste und/oder das zweite Rahmenteil vorzugsweise mit Stabilisierungsrippen versehen werden.

Der Spritzenkörper wird vorzugsweise aus einem Kunststoff gefertigt, der autosterilisierende Eigenschaften aufweist oder mit Mitteln beschichtet ist, die autosterilisierende Eigenschaften aufweisen, wie z.B. Thymol.

Erfindungsgemässe Einwegspritzen können kostengünstig im Nutzen, d.h. im Verbund von mehreren Einheiten gefertigt und ausgeliefert werden. Es werden daher Verbundschalen vorgesehen, welche zahlreiche einzelne Schalen umfassen.

Nach der Fertigung sind daher mehrere Einwegspritzen parallel und/oder seriell miteinander verbunden. Diese Verbindungen werden vorzugsweise ebenfalls als Sollbruchstellen ausgestaltet, so dass einzelne Einwegspritzen oder Gruppen von Einwegspritzen wahlweise aus dem Verbund gelöst werden können. Der Anwender kann daher eine Serie von miteinander verbundenen Einwegspritzen kaufen, die beispielsweise ein Band bilden, das gerollt werden kann. Von diesem Band können jeweils mehrere für den täglichen Bedarf erforderliche Einwegspritzen abgetrennt werden.

Die erfindungsgemässen Einwegspritzen können universell eingesetzt werden. Die Einwegspritzen eignen sich für die Selbstmedikation, aber auch für den Notfalleinsatz des Arztes.

Nachfolgend wird die Erfindung anhand von Zeichnungen näher erläutert. Dabei zeigt:
- Fig. 1a: die Frontseite einer erfindungsgemässen Einwegspritze 1, die einen Spritzenkörper 10 mit einem ersten Rahmenteil 11 umfasst, welcher einen Behälter 110 für eine medizinische Flüssigkeit 2 und eine darin eingesetzte Injektionsnadel 13 umfasst, die in eine Ausnehmung 120 in einem zweiten Rahmenteil 12 hinein ragt und dort vor mechanischen Einwirkungen geschützt ist;
- Fig. 1b: die Einwegspritze 1 von Figur 1a von der Seite gezeigt;
- Fig. 2a: die Einwegspritze 1 von Figur 1a bei der Vorbereitung für den Einsatz durch Entfernen des zweiten Rahmenteils 12;
- Fig. 2b: den Einsatz der Einwegspritze 1 von Figur 1a nach Entfernung des zweiten Rahmenteils 12; und
- Fig. 2c: das von der Einwegspritze 1 entfernte zweite Rahmenteil 12.

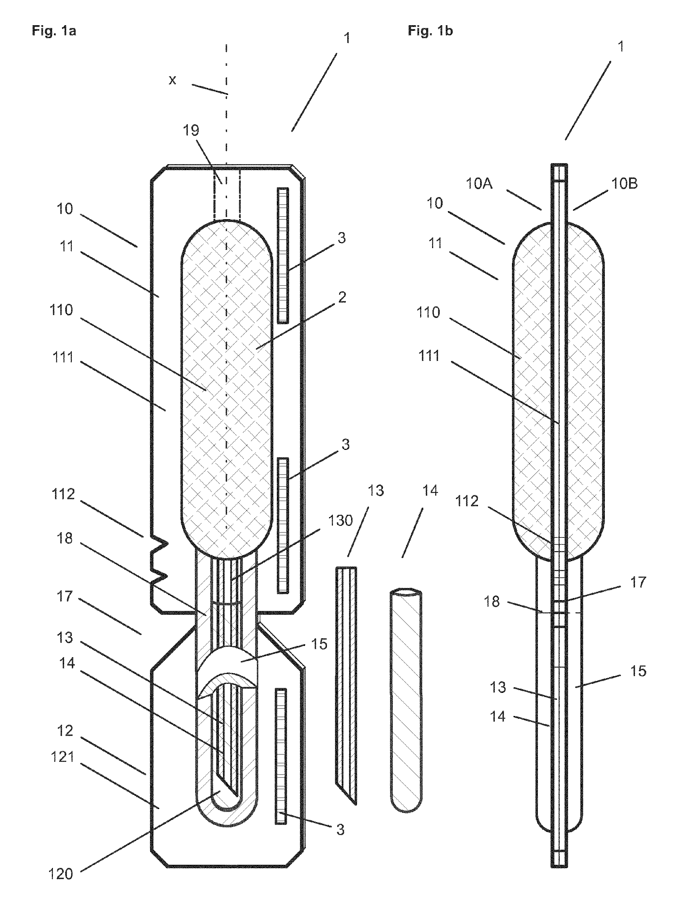

Die Figuren 1a und 1b zeigen eine erfindungsgemässe Einwegspritze 1 von vorn und von der Seite. Die Einwegspritze 1 weist einen Spritzenkörper 10 auf, der ein erstes und ein zweites Rahmenteil 11, 12 umfasst, die über eine Sollbruchstelle 18 einstückig miteinander verbunden sind.

Das erste und das zweite Rahmenteil 11, 12 sind durch ein tunnelförmiges Verbindungselement 15 miteinander verbunden, das eine Ausnehmung 120 umschliesst, die der Aufnahme der Injektionsnadel 13 dient. Das in Schnittdarstellung gezeigte Verbindungselement 15 erstreckt sich in das zweite Rahmenteil 12 hinein und ist frontseitig abgeschlossen.

Durch die tunnelartige Ausgestaltung des Verbindungselements 15 wird die Injektionsnadel 13 umschlossen und vollständig geschützt. Möglich ist hingegen die Reduktion des Verbindungselements 15 auf zwei Stege, welche die Ausnehmung 120 links und rechts begrenzen, wie dies in der Schnittdarstellung gezeigt ist. Die Tunnelüberdeckung ist in diesem Fall weggeschnitten und kann durch eine widerstandsfähige Folie ersetzt werden.

Das erste Rahmenteil 11 umfasst einen Rahmen 111, der einen komprimierbaren Behälter 110 umschliesst. Dazu wird der Behälter 110 mit einer relativ dünnen Kunststoffwand gefertigt, die auch als Folie ausgebildet sein kann. Der Rahmen 111 und die Wand des Behälters 110 bilden vorzugsweise eine Einheit. Der Unterschied der Wanddicken des Rahmens 111 und des Behälters 110, der beispielsweise bei einem Faktor im Bereich von 5-20 liegt, wird beispielsweise dadurch erzielt, dass die Behälterwand unter thermischer Einwirkung nach aussen gedehnt wird. Alternativ können auch Rohlinge vorgesehen werden, bei denen im Bereich des Behälters geringe Wanddicken vorliegen. Bei einer Dehnung der Behälterwand nach aussen erfolgt daher eine weitere Reduktion des Wanddurchmessers. Sofern Giessverfahren eingesetzt werden, werden die gewünschten Formen und Wanddicken anhand entsprechend ausgestalteter Giessformen erzielt.

Anschliessend an den Behälter 110 ist eine Injektionsnadel 13 vorgesehen, deren eines Endstück 130, welches dem Behälter 110 zugewandt ist und gegebenenfalls in diesen hinein ragt, vom ersten Rahmenteil 11 dicht umschlossen ist. Die Injektionsnadel 13 ist vom ersten Rahmenteil 11, anschliessend an den Behälter 110, stabil gehalten. Die Längsachsen des Behälters 110 und der Injektionsnadel 13 sind vorzugsweise koaxial entlang einer gemeinsamen Achse x ausgerichtet.

Die Injektionsnadel 13, die eine übliche Länge von einigen Zentimetern aufweist, ragt in eine Ausnehmung 120 hinein, die im zweiten Rahmenteil 12 vorgesehen ist. Wie erwähnt wird vorzugsweise ein Tunnel 15 vorgesehen, der das erste und das zweite Rahmenteil 11, 12 miteinander verbindet und der vorzugsweise an den Behälter 110 anschliesst.

Das zweite Rahmenteil 12 weist einen mit der Ausnehmung 120 versehen Rahmen 121 und/oder einen Tunnel 15 auf, der die Injektionsnadel 13 umfasst und schützt. Mechanische Einwirkungen auf die noch nicht benutzte Einwegspritze 1 werden daher durch den Spritzenkörper 10, insbesondere durch das zweite Rahmenteil 12 und den Tunnel 15 aufgefangen und können nicht zu einer schädlichen Deformation des Behälters 110 und der Injektionsnadel 13 führen.

Das erste und das zweite Rahmenteil 11, 12 sind über das Verbindungselement 15 miteinander verbunden, das derart mit einer Sollbruchstelle 18 versehen ist, dass diese durch manuelle Zugkräfte und Biegekräfte nicht voneinander getrennt werden können. Erst durch eine Drehung des zweiten Rahmenteils 12 gegenüber dem ersten Rahmenteil 11 kann die Sollbruchstelle 18, aufgebrochen werden. Dazu weist die Sollbruchstelle 18 Materialverdünnungen und/oder Perforation und vorzugsweise seitliche Einrisse auf, die sich durch gegenseitige Drehung der Rahmenteile 11, 12 leicht erweitern lassen. Damit der Kraftaufwand zum Auftrennen der Sollbruchstelle 18 klein gehalten werden kann, wird in deren Bereich ein reduzierter Querschnitt vorgesehen. Dazu werden im Bereich der Verbindung zwischen dem ersten und zweiten Rahmenteil 11, 12 vorzugsweise Einschnitte 17 vorgesehen. Aufgrund der grösseren Breite des zweiten Rahmenteils 12 im Vergleich zur Sollbruchstelle 18 wirkt dieses wie eine Flügelmutter, weshalb grössere Drehmomente auf die Sollbruchstelle 18 übertragen werden können. Somit können auch ältere und schwächere Personen die beiden Rahmenteile 11, 12 leicht voneinander trennen.

Zur Entkopplung und weiteren Stabilisierung der Injektionsnadel 13 wird vorzugsweise eine Schutzhülle 14 auf diese aufgesetzt, die vorzugsweise ebenfalls im ersten Rahmenteil 11 verankert ist. Die Schutzhülle 14 verleiht der Injektionsnadel 13 einen mechanischen Schutz und schliesst diese frontseitig vorzugsweise dicht ab. Flüssigkeit 2 kann daher nicht aus den Behälter 110 durch die Injektionsnadel 13 austreten. Ferner isoliert die Schutzhülle 14 die Injektionsnadel 13 gegen das zweite Rahmenteil 12, wodurch verhindert wird, dass diese beispielsweise während des Fertigungsprozesses miteinander verkleben können. Während des Fertigungsprozesses kann daher sogar eine Gussmasse appliziert werden, mittels der die Schutzhülle 14 im zweiten Rahmenteil 12 eingegossen und fixiert wird. Somit wird sichergestellt, dass die Injektionsnadel 13 sicher und stabil gelagert ist und dass beim Drehen des zweiten Rahmenteils 12 keine Kräfte auf die Injektionsnadel 13 übertragen werden.

In Figur 1a sind in Schnittdarstellung eine Injektionsnadel 13 und eine Schutzhülle 14 auch alleinstehend gezeigt. Zum weiteren Schutz der Einwegspritze 1 wird der Spritzenkörper 10 oder wenigstens das zweite Rahmenteil 12 mit einer Schutzfolie überdeckt, die vom Anwender leicht gelöst werden kann.

Es ist ersichtlich, dass die erfindungsgemässe Einwegspritze 1 einfach, kompakt und robust aufgebaut ist. Zur weiteren Stabilisierung der Einwegspritze 1 wird der Spritzenkörper 10 vorzugsweise zusätzlich mit Stabilisierungsrippen 3 versehen. Mit nur wenig zusätzlichem Material kann dadurch eine deutliche Steigerung der Festigkeit erzielt werden.

Die Fertigung der Einwegspritzen 1 erfolgt vorzugsweise im Nutzen, d.h. es werden mehrere Einwegspritzen 1 im Verbund gefertigt.

Figur 1b zeigt, dass der Spritzenkörper 10 vorteilhaft aus zwei vorzugsweise identischen Schalen 10A, 10B zusammengesetzt werden kann. Dabei kann vorgesehen werden, dass bei der Verbindung der beiden Schalen 10A, 10B ein Einfüllkanal 19 geöffnet bleibt, durch den die medizinische Flüssigkeit 2 in den Behälter 110 eingefüllt werden kann. Nach dem Einfüllen der Flüssigkeit 2 wird der Einfüllkanal 19 z.B. durch Verschweissen oder Einsetzen eines Füllelements verschlossen.

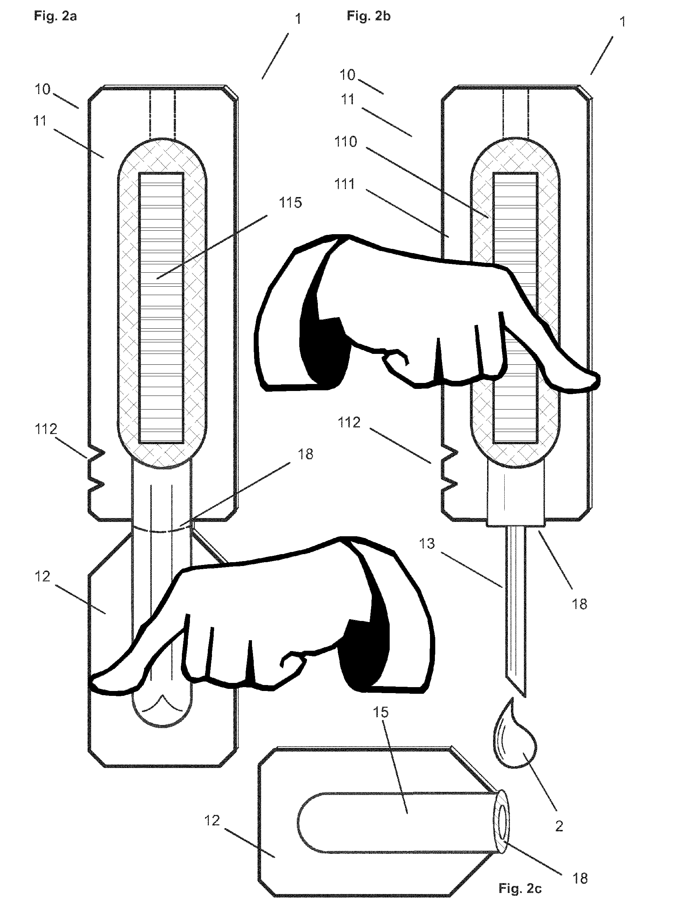

Die im Nutzen gefertigten Einwegspritzen 1 können im Verbund verpackt und ausgeliefert werden. Der Anwender kann daher einen Verbund von gefüllten Einwegspritzen 1 kaufen und davon die für den täglichen Bedarf erforderliche Menge abtrennen. Beispielsweise enthält eine Packung einen Verbund von 3x7 Einwegspritzen 1. Der Anwender kann daher drei miteinander verbundene Einwegspritzen 1 aus dem Verbund lösen und als tägliche Ration mit sich tragen. Aus dem Teilverbund von drei Stück wird der Anwender z.B. jeweils zwei Stück für die Anwendung am Morgen und am Nachmittag lösen und ein Stück als Reserve behalten. Auf diese Weise gelingt eine effiziente und wirtschaftliche Fertigung der Einwegspritzen 1 und eine vorteilhafte Anwendung derselben, die in den Figuren 2a und 2b illustriert ist.

In Figur 2a ist gezeigt, dass das zweite Rahmenteil 12 manuell erfasst gedreht und vom ersten Rahmenteil 11 gelöst wird. In der Folge verbleiben das in Figur 2b gezeigte erste Rahmenteil 11 mit der Injektionsnadel 13 und das in Figur 2c gezeigte zweite Rahmenteil 12, welches an der Sollbruchstelle 18 vom ersten Rahmenteil 11 getrennt wurde. Durch Druck auf den Behälter 110 kann nun Flüssigkeit 2 aus dem Behälter 110 gepresst und durch die Injektionsnadel 13 nach aussen gefördert werden. Damit der Inhalt bequem und vollständig aus dem Behälter 110 gepresst werden kann, ist dieser in der gezeigten Ausgestaltung mit balkenförmigen Greifelementen 115 versehen, welche den gesamten Behälter 110 überdecken. Die Flüssigkeit 2 kann daher nicht zwischen den Fingern entweichen, sondern wird mittels der Greifelemente 115 praktisch vollständig aus dem Behälter 110 verdrängt. Die beiden Greifelemente 115 werden dabei zusammengedrückt, bis sie auf einander auftreffen. Der Anwender kann dadurch erkennen, dass die Applikation der Flüssigkeit 2 abgeschlossen ist und die Einwegspritze 1 entfernt und entsorgt werden kann.

In Figur 2a ist ferner gezeigt, dass der Spritzenkörper 10 mit Einformungen 112 versehen ist, die der Kennzeichnung der Einwegspritzen 1 dienen. Ein Anwender kann daher durch Abtasten der Einwegspritze 1 überprüfen, ob er die richtige Einwegspritze ausgewählt hat.

## Patentansprüche

**1.** Einwegspritze (1) mit einem Spritzenkörper (10), der einen der Aufnahme einer Flüssigkeit (2) dienenden Behälter (110) umfasst, und mit einer Injektionsnadel (13), durch die hindurch die Flüssigkeit (2) aus dem Behälter (110) transferierbar ist, **dadurch gekennzeichnet, dass** der Spritzenkörper (10) ein erstes Rahmenteil (11) aufweist, das mit dem manuell komprimierbaren Behälter (110) versehen ist und das über wenigstens eine Sollbruchstelle (18) einstückig mit einem zweiten Rahmenteil (12) verbunden ist, welches eine Ausnehmung (120) aufweist, in die die Injektionsnadel (13) hinein ragt, deren dem Behälter (11) zugewandtes Endstück (130) vom ersten Rahmenteil (11) dicht abschliessend gehalten ist.

**2.** Einwegspritze (1) nach Anspruch 1, **dadurch gekennzeichnet, dass** das erste und das zweite Rahmenteil (11, 12) durch ein Verbindungselement (15), wie einen oder mehrere Stege oder durch einen frontseitig abgeschlossenen Tunnel, einstückig miteinander verbunden sind, wobei das Verbindungselement (15) die wenigstens eine Sollbruchstelle (18) aufweist und die Ausnehmung (120) zumindest teilweise begrenzt.

**3.** Einwegspritze (1) nach Anspruch 1 oder 2, **dadurch gekennzeichnet, dass** der Spritzenkörper (10) aus einem Kunststoff gefertigt ist, der vorzugsweise autosterilisierende Eigenschaften aufweist oder der mit autosterilisierendem Material beschichtet ist und dass der Spritzenkörper (10) vorzugsweise mit Rippen (3) versehen ist, welche die Stabilität des Spritzenkörpers (10) erhöhen.

**4.** Einwegspritze (1) nach Anspruch 1, 2 oder 3, **dadurch gekennzeichnet, dass** der Spritzenkörper (10) aus zwei zueinander komplementären und miteinander verbundenen, gegebenenfalls miteinander verklebten oder verschweissten Schalen (10A, 10B), besteht.

**5.** Einwegspritze (1) nach einem der Ansprüche 1 - 4, **dadurch gekennzeichnet, dass** der Behälter (110) aus elastischem Material oder aus einer Folie besteht und/oder dass der Behälter (110) einen vorzugsweise kreisförmigen oder elliptischen Querschnitt aufweist.

**6.** Einwegspritze (1) nach Anspruch 5, **dadurch gekennzeichnet, dass** der Behälter (110) auf den einander gegenüberliegenden Seiten je mit einem starren Greifelement (115) versehen ist.

**7.** Einwegspritze (10) nach einem der Ansprüche 1 - 6, **dadurch gekennzeichnet, dass** die Injektionsnadel (13) von einer Schutzhülle (14) umschlossen ist, welche die Injektionsnadel (13) dicht abschliesst und/oder gegenüber dem zweiten Rahmenteil (12) isoliert.

**8.** Einwegspritze (1) nach Anspruch 7, **dadurch gekennzeichnet, dass** die Schutzhülle (14) im zweiten Rahmenteil (12) eingegossen ist.

**9.** Einwegspritze (1) nach einem der Ansprüche 1 - 8, **dadurch gekennzeichnet, dass** der Spritzenkörper (10) von einer Metallfolie oder einer Kunststofffolie umschlossen ist.

**10.** Einwegspritze (1) nach einem der Ansprüche 1 - 9, **dadurch gekennzeichnet, dass** die Sollbruchstelle (18) durch Materialbeeinträchtigungen, wie eine Materialverdünnung, Perforationen oder Einrisse, gebildet wird.

**10.** Einwegspritze (1) nach einem der Ansprüche 1 - 9, **dadurch gekennzeichnet, dass** zwischen dem ersten und dem zweiten Rahmenteil (11, 12) vorzugsweise keilförmige Einschnitte (17) vorgesehen sind, die an die Sollbruchstelle (18) anschliessen.

**11.** Einwegspritze (1) nach einem der Ansprüche 1 - 10, **dadurch gekennzeichnet, dass** das erste Rahmenteil (11) den Behälter (110) umschliesst und dass das zweite Rahmenteil (12) die Injektionsnadel (13) umschliesst, die gegebenenfalls im tunnelförmigen Verbindungselement (15) angeordnet ist, welches sich in das zweite Rahmenteil (12) hinein erstreckt.

**13.** Einwegspritze (1) nach Anspruch 12, **dadurch gekennzeichnet, dass** das erste und/oder das zweite Rahmenteil (11, 12) eine durch Einformungen gebildete und somit abtastbare und/oder farbig dargestellte Kennzeichnung aufweist.

**14.** Einwegspritze (1) nach einem der Ansprüche 1 - 13, **dadurch gekennzeichnet, dass** die Einwegspritzen im Nutzen gefertigt sind.

**15.** Einwegspritze (1) nach Anspruch 14, **dadurch gekennzeichnet, dass** mehrere Einwegspritzen (1) parallel und/oder seriell über weitere Sollbruchstellen miteinander verbunden sind.
